# EUROPEAN PATENT SPECIFICATION

(11) **EP 3 801 061 B1**
(45) Date of publication and mention of the grant of the patent: **16.08.2023**
(21) Application number: 19727047.3
(22) Date of filing: 03.06.2019
(51) Int. Cl.: A23L 33/105, A23L 29/00, A23L 33/135, A61K 45/06, A61K 35/66, A61K 36/00, G01N 33/50, A23L 29/30, A23L 33/10, A61K 36/23, A61K 36/28, A61K 36/48, A61K 36/52, A61K 36/60, A61K 36/752, A61K 36/889, A61K 36/8962, A61K 36/899

(54) **MICROVESICLES DERIVED FROM FERMENTED PLANT-BASED PRODUCTS, METHOD FOR THEIR PREPARATION AND USE**
MIKROVESIKEL AUS FERMENTIERTEN PFLANZENPRODUKTEN, VERFAHREN ZU DEREN HERSTELLUNG UND VERWENDUNG
MICROVESICULES DERIVES DE PRODUITS FERMENTESCIBLES A BASE DE PLANTES, LEUR PROCEDE DE PREPARATION ET LEUR UTILISATION

(30) Priority: 01.06.2018 EP 18175575
(43) Date of publication of application: 14.04.2021
(73) Proprietor: NZYM Deutschland GmbH, 85662 Hohenbrunn (DE)
(72) Inventor: MAY, Alexander, 9057 Weissbad (CH)
(74) Representative: Steinecke, Peter
(86) International application number: PCT/EP2019/064370
(87) International publication number: WO 2019/229271

(56) References cited:
- EP-A2- 3 269 378
- EP-B1- 1 153 549
- EP-B1- 2 839 278
- WO-A1-2012/093755
- WO-A1-2017/052271
- KR-A- 20160 032 722
- KR-A- 20180 018 354
- KR-A- 20180 019 482
- YASUSHI TANAKA ET AL: "Monitoring of the microbial communities involved in the soy sauce manufacturing process by PCR-denaturing gradient gel electrophoresis", FOOD MICROBIOLOGY., vol. 31, no. 1, 23 February 2012 (2012-02-23), pages 100-106, XP055526752, GB ISSN: 0740-0020, DOI: 10.1016/j.fm.2012.02.005
- SONGWEN JU ET AL: "Grape Exosome-like Nanoparticles Induce Intestinal Stem Cells and Protect Mice From DSS-Induced Colitis", MOLECULAR THERAPY : THE JOURNAL OF THE AMERICAN SOCIETY OF GENE THERAPY, vol. 21, no. 7, July 2013 (2013-07), pages 1345-1357, XP055300276, US ISSN: 1525-0016, DOI: 10.1038/mt.2013.64
- Yu Siran ET AL: "Fermentation Results in Quantitative Changes in Milk-Derived Exosomes and Different Effects on Cell Growth and Survival", Journal of Agricultural and Food Chemistry, vol. 65, no. 6, 13 January 2017 (2017-01-13), pages 1220-1228, XP055909749, US ISSN: 0021-8561, DOI: 10.1021/acs.jafc.6b05002

## Description

### FIELD OF THE INVENTION

The present invention generally relates to extracellular vesicles derived from the fermentation product of natural raw materials by microorganisms. In particular, the present invention relates to extracellular vesicles derived from a fermentation product of plant raw materials, methods of preparation or enrichment, as well as compositions containing extracellular vesicles of the invention. Furthermore, the present invention relates to the use of the extracellular vesicles and compositions of the invention in the manufacture of foods, food supplements and in cosmetic or personal care products, as well as to the use as a drug.

### BACKGROUND OF THE INVENTION

Foods, especially those based on plants such as fruits and medicinal herbs, contain ingredients that are known for their positive effect on general health and, for example, have anti-inflammatory effects or can serve as active ingredients for the treatment or prevention of diseases. However, due to increasingly stressful daily routines and a lack of time, more and more people are now turning to synthetic dietary supplements, vitamin and mineral preparations, as well as instant products to compensate for their unhealthy lifestyle.

The majority of dietary supplements available in pharmacies, drugstores and supermarkets consist of synthetically produced vitamins and isolated minerals and trace elements, which often have negative health effects. On the one hand, synthetically produced substances are less tolerable than natural preparations and on the other hand artificial preparations often contain harmful additives and chemicals. Thus, in recent years natural products have become more and more popular as dietary supplements for health promotion, but also as classic foods or in the cosmetics industry and there is an increasing demand for these. There is also a steadily increasing demand for "natural" drugs, for example drugs made from plant ingredients, for the treatment of diseases. Thus, natural products are becoming increasingly important. Natural products are products such as materials, mixtures of substances or foods or food supplements which are largely natural or made from naturally occurring raw materials.

WO 2012/093755 A1 and KR 2016 0032722 A describe such natural products, in particular extracellular vesicles from soy sauce or soy paste as well as from lactic acid beverages. Therefore, it is the object of the present invention to provide a further product derived from natural raw materials that meets the above-mentioned needs. According to the invention, this task is solved by the embodiments characterized in the claims and the description.

### SUMMARY OF THE INVENTION

The present invention relates to the embodiments as characterized in the claims and in particular to microvesicles, *i.e.* extracellular vesicles from the product of the fermentation of natural raw materials by microorganisms, wherein the natural raw materials are plant raw materials and comprise lemons, pitted dates, figs, walnuts, coconuts, soybeans, onions, mung bean sprouts, celery, artichokes, millet, and peas, and wherein the extracellular vesicles (EV) are of plant and microbial origin, wherein the fermentation of the natural raw materials comprises
(a) the production of a first ferment extract or ferment powder;
(b) subjecting a portion of the first ferment extract or ferment powder to at least one further fermentation; and
(c) mixing the portion/s of the further fermentation/s with the remaining portion of the first fermentation extract or fermentation powder,
wherein the microorganisms consist of Lactobacillus, and wherein the size of the EV is between 150-210 nm, 240-300 nm, 350-410 nm and 575-635 nm, preferably between 175-185 nm, 265-275 nm, 375-385 nm and 600-610 nm, and wherein the size of the EV is measured by nanoparticle tracking analysis, a method which is described in Example 2 in more detail. In particular, the present invention relates to extracellular vesicles derived from said fermentation product of natural raw materials which are suitable as food for humans, for example directly as fruit and/or in fermented form. In particular, the present invention relates to extracellular vesicles as characterized in the claims derived from the fermentation product of plant raw materials, methods for their preparation or enrichment and compositions containing extracellular vesicles of the invention. Furthermore, the present invention relates to the use of the extracellular vesicles and compositions of the invention as characterized in the claims in the manufacture of foods, food supplements and/or in cosmetic or personal care products, as well as to the use as drugs.

Fermented foods have a positive effect on health and can even prevent and cure diseases, *inter alia* through the probiotic bacteria they contain. However, it was not known until now that such fermentation products have numerous extracellular vesicles. Surprisingly, this could be shown in the course of experiments on the fermentation of natural raw materials; see the Examples.

As demonstrated in the Examples the present invention *inter alia* relates to EV derived from a fermentation product of plant raw material and microorganisms, characterized in that the EV are of plant and microbial origin, wherein the plant raw materials comprise lemons, pitted dates, figs, walnuts, coconuts, soybeans, onions, mung bean sprouts, celery, artichokes, millet, and peas, wherein the fermentation of the natural raw materials comprises
(a) the production of a first ferment extract or ferment powder;
(b) subjecting a portion of the first ferment extract or ferment powder to at least one further fermentation; and
(c) mixing the portion/s of the further fermentation/s with the remaining portion of the first fermentation extract or fermentation powder,
wherein the microorganisms consist of *Lactobacillus,* and wherein the size of the EV is between 150-210 nm, 240-300 nm, 350-410 nm and 575-635 nm, preferably between 175-185 nm, 265-275 nm, 375-385 nm and 600-610 nm, and wherein the size of the EV is measured by nanoparticle tracking analysis, a method which is described in Example 2 in more detail.

In a further embodiment, the vesicles according to the invention originate from a fermentation product, whereby this product or the raw material additionally comprises extracts, concentrates, vitamins, minerals, trace elements, flavoring substances and/or further organic compounds.

In another embodiment, the vesicles of the invention originate from a fermentation product, wherein the raw material and/or fermentation product is free or substantially free of coloring agents, preservatives, alcohol, sugar, gluten and milk.

The size of the vesicles of the invention is between 150 and 210 nm, between 240 and 300 nm, between 350 and 410 nm and 575 and 635 nm. More preferably, the size of the vesicles of the invention is between 160 and 200 nm, between 250 and 290 nm, between 360 and 400 nm and between 585 and 625 nm. Even more preferably, the size of the vesicles of the invention is between 170 and 190 nm, between 260 and 280 nm, between 370 and 390 nm and between 595 and 615 nm. Most preferably, the size of the vesicles of the invention is between 175 and 185 nm, between 265 nm and 275 nm, between 375 and 385 nm and between 600 and 610 nm.

Furthermore, the vesicles of the invention comprise an active ingredient of interest, preferably selected from the group consisting of a phytochemical active ingredient, nucleic acid molecules such as ncRNA, rRNA, mRNA, miRNA, tRNA, dsDNA and/or sDNA, proteins, in particular enzymes, lipids and membrane receptors.

A further embodiment of the present invention relates to a method for the preparation or enrichment of the vesicles of the invention, wherein the method comprises the fermentation of the raw materials in the presence of microorganisms described herein and the recovery or enrichment of the vesicles of the invention from the fermentation product. As described above, fermentation of the raw materials comprises the production of a first ferment extract or ferment powder, subjecting a portion of the first ferment extract or ferment powder to at least one further fermentation, and mixing that portion with the remaining portion of the first ferment extract or ferment powder. This fermentation method is also referred to as cascade fermentation as described in European patents EP 1 153 549 B1 and EP 2 560 506 B1.

In another embodiment of the method of the invention, plant raw material containing the active ingredient of interest is fermented or the active ingredient is added to the raw material and/or fermentation product.

According to the invention, the described fermentation is carried out by *Lactobacillus.*

In one embodiment of the present invention, the enrichment or isolation of the vesicles of the invention is carried out by means of ultra- and density gradient centrifugation, respectively, electrophoresis, chromatography, column method, precipitation, lyophilization, drying and/or nanofiltration.

A further embodiment of the present invention relates to a composition containing the vesicles of the invention, wherein their concentration is preferably greater than 1 × 10⁸ /ml, particularly preferably greater than 1 × 10⁹ /ml and particularly preferably greater than 1 × 10¹⁰ /ml.

The composition of the invention is preferably in liquid, powder or lyophilized form and is a natural product, food for special medical purposes (balanced diets), dietary food, food supplement, cosmetic or body care product, in particular a lotion. The composition of the invention may also comprise a pharmaceutically or physiologically acceptable carrier and/or stabilizer.

The present invention also relates to the use of the vesicles or the composition of the invention in the manufacture of foods for special medical purposes (balanced diets), dietary foods, food supplements and/or in cosmetic or personal care products. Furthermore, the present invention relates to the vesicles or the composition of the invention for use as a drug.

### BRIEF DESCRIPTION OF THE DRAWINGS

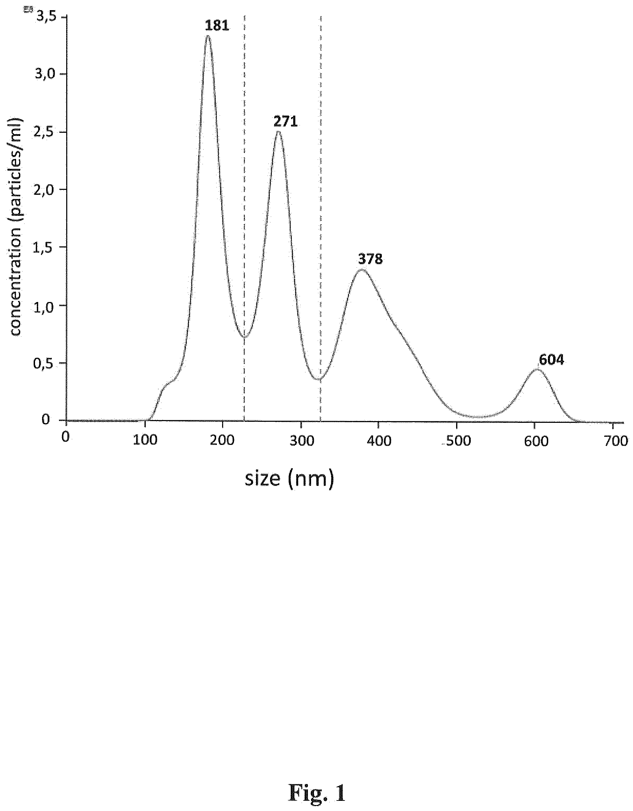

- Fig. 1:: NanoSight^{™} measurement of the size of extracellular vesicles isolated from a fermentation product of plant raw materials. The isolation was performed using the ExoQuick^{™} method and the software "Nanoparticle Tracking Analysis" (NTA) was used. The results of a representative experiment are shown here. Extracellular vesicles of four different sizes were isolated, with sizes of 181 nm, 271 nm, 378 nm and 604 nm.

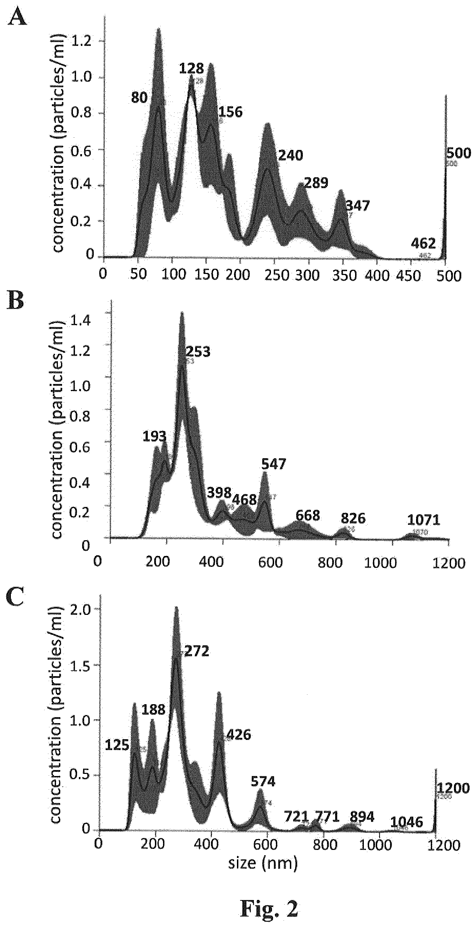

- Fig. 2:: NanoSight^{™} measurement of the size of extracellular vesicles isolated from squeezed lemon juice (A), fermented *Lactobacillus paracasei* (with fructose) (B) and fermented lemons *Lactobacillus paracasei* (7-day single fermentation under lab conditions) (C).

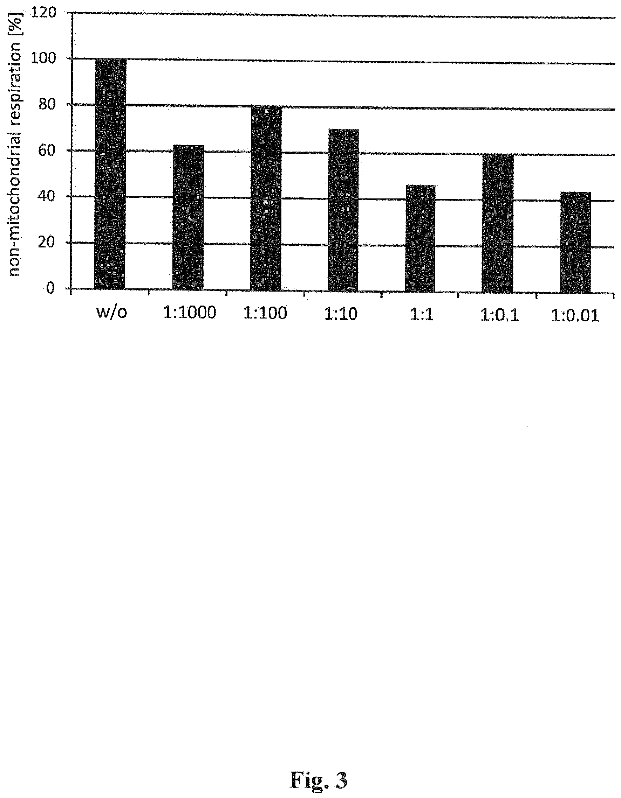

- Fig. 3:: Analysis of the non-mitochondrial oxygen consumption rate (OCR) of PBMC treated with different concentrations of the extracellular vesicles of the invention. The proportion of non-mitochondrial respiration on the overall respiration of untreated cells (w/o) is set to 100%. The data indicated that treatment of the cells with extracellular vesicles reduces non-mitochondrial respiration.

### DEFINITIONS

Initially, some of the terms used in this application will be explained in the following in more detail: "Bioactive substances" or "biologically active substances" are substances in foodstuffs which do not have a nutrient character but have a health-promoting effect. These are primarily secondary phytochemicals, but also dietary fibres and substances in fermented foods.

Extracellular vesicles are nanoscale membrane vesicles that are secreted by most cell types *in vivo* and *in vitro* into the environment and can be reabsorbed by a large number of cells. In the sense of the present invention, the term "extracellular vesicles" as used herein includes exosomes, microvesicles, apoptotic bodies as well as vesicle or exosome-like nanoparticles and nanovesicles. Exosomes usually have a diameter of only 50 nm to 150 nm, followed by microvesicles from 100 nm to 1000 nm. Even larger are the apoptotic bodies with diameters ranging from 500 nm to 4000 nm, which are released during programmed cell death by the constriction of entire cell parts. Extracellular vesicles also include exosomes, epididimosomes, argosomes, exosome-like vesicles, microparticles, promininosomes, prostasomes, dexosomes, texosomes, and oncosomes.

Microvesicles are formed by protrusion of the outer cell membrane, exosomes by invagination of so-called endosomes, which belong to the endosomal membrane system inside the cell - as lysosomes (intracellular digestion) or the endoplasmic reticulum (membrane biosynthesis). These endosomes can form "vesicles in vesicles" through membrane invaginations. This results in multivesicular bodies (MVB) that fuse with the outer cell membrane and release their contents as exosomes into the extracellular space. The vesicles contain various biomolecules of their cell of origin, including nucleic acids (mRNA, miRNA), proteins, lipids, polysaccharides or other bioactive substances of varying composition, and serve as transport vehicles and for the secretion of cell components. The apoptotic bodies can also contain DNA, histones and organelles.

"Fermentation" or "fermenting" according to the present invention means the microbial or enzymatic conversion of organic matter into acid, gas or alcohol. Fermentation is deliberately used in biotechnology. This is done either by adding bacterial, fungal or other biological cell cultures or by adding enzymes (ferments), which carry out the fermentation within the framework of their enzyme-catalyzed metabolism. Some of these microorganisms are already naturally present in the starting materials, for example during spontaneous fermentation.

Unless otherwise specifically defined or used, the term "fermentation product" shall mean any product or intermediate resulting from the conversion of biological materials, in particular natural raw materials with the aid of bacterial, fungal or cell cultures, in liquid or solid form, and containing the same.

"Natural products" refer to products such as materials, mixtures of substances or foodstuffs or food supplements which are largely natural or made from naturally occurring raw materials.

As used herein, the term "phytochemical active ingredient" refers to a non-nutritious plant-based compound. Phytochemical substances are also known as phytonutrients or secondary phytochemicals that make up the plant's immune system. Examples of phytochemical ingredients include monophenols, flavonoids such as flavonols, flavanones, flavones, flavan-3-ols, anthocyanins, anthocyanidins, isoflavones, dihydroflavonols, chalcones and cumestanes, phenolic acids, hydroxycinnamic acids, lignans, tyrosol esters, stilbenoids, hydrolysable tannins, carotenoids such as carotenes and xanthophylls, monoterpenes, saponins, lipids such as phytosterols, tocopherols and omega-3,6,9 fatty acids, diterpenes, triterpinoids, betalains such as betacyanins and betaxanthins, dithiolthiones, thiosulfonates, indoles and glucosinolates. However, those examples are not to be regarded as limiting.

### DETAILED DESCRIPTION OF THE INVENTION

In the course of experiments on the fermentation of natural raw materials, it was surprisingly shown that extracellular vesicles are present in a fermentation product made from plant raw materials. As described below and illustrated in the Examples, the vesicles of the invention were surprisingly detected and isolated from a certain batch of the Regulat products of Dr. Niedermaier Pharma GmbH, a series of multiple fermented food supplements, wherein the REGULATESSENZ^{®} is the basic ingredient of these products; see also Example 1. Although the principle of manufacturing the Regulat products, e.g. cascade fermentation, is described in general terms in the two European patents EP 1 153 549 B1 and EP 2 560 506 B1 together with parts of the ingredients, the present invention now enables for the first time a quality to control be carried out so that the Regulat products and equivalent products based on the fermentation of natural raw materials which are beneficial to health can be manufactured in constant quality by controlling and monitoring the quantity and quality of the extracellular vesicles as described above and illustrated in the Examples. Furthermore, the vesicles available from such fermentation products may advantageously be used themselves in health-promoting and general well-being products as described below.

Extracellular vesicles are nanoscale membrane vesicles that are secreted by most cell types *in vivo* and *in vitro* into the environment. The vesicles contain various biomolecules of their cell of origin, including nucleic acids such as mRNA, miRNA or siRNA, proteins, lipids, polysaccharides and other bioactive ingredients of varying composition, and serve as transport vehicles and for the secretion of cell components.

For example, such vesicles are released from the surface of many different human cell types into various body fluids such as plasma, milk, saliva, sweat, tears, sperm and urine. In the pharmaceutical industry, extracellular vesicles are currently mainly used as vesicles with artificially loaded contents, for example to transport active pharmaceutical ingredients in the human body to their destination or to increase the bioavailability of substances.

In contrast, the vesicles of the invention have natural ingredients or health-promoting active ingredients and can be obtained from a fermentation product of plant raw materials and used as a food supplement or added to foods, food supplements, cosmetics or body care products and also formulated as a drug.

The vesicles of the invention thus offer a solution for the constantly growing need for natural compounds for general health promotion as well as for the prevention and treatment of diseases. Accordingly, the isolation of these vesicles from natural raw materials, in particular from plant raw materials, holds great potential for various industries, in particular for the food and health industry.

With regard to the aspect of renewable raw materials and the protection of the environment as well as the already known positive effect of the fermentation product, which served as a basis for the isolation of the vesicles of the invention in the Example performed in accordance with the present invention, and the associated properties of the vesicles originating from this product, the present invention, relates to extracellular vesicles, which are obtainable from a fermentation product of plant raw materials as characterized in the claims.

Thus, the present invention provides extracellular vesicles (EV) derived from a fermentation product of plant raw material as characterized in the claims. According to the present invention, extracellular vesicles, also referred to as membrane vesicles or outer membrane vesicles in the case of Gram-negative bacteria, include exosomes, microvesicles, apoptotic bodies as well as vesicle- or exosome-like nanoparticles, and nanovesicles. As already described above, the different types of vesicles differ *inter alia* in their size, which enables an expert to distinguish them, for example by means of the procedure described in Example 2.

Furthermore, the publication of Müller, J Bioanal Biomed 4 (2012), 4 summarizes a series of methods for the validation of the enrichment of extracellular vesicles from a cell population. These include several parameters such as size and density to differentiate between exosomes, microvesicles and apoptotic bodies themselves as well as to distinguish contaminating entities, lipids (droplets), lipoproteins, protein aggregates, protein-phospholipid micelles, cells and membrane fragments.

An overview of the use of the physico-chemical and biochemical properties of extracellular vesicles and techniques for their isolation is, for example, provided by Li et al., Theranostics 7 (2017), 789-804. In addition, a consolidation of the prior art for the isolation of extracellular vesicles, including exosomes, including indication of the properties of the individual methods, including the size of the isolated vesicles depending on the method, isolation efficiency, vesicle yield, properties of isolated extracellular vesicles and workload is provided by Konoshenko et al., in BioMed Research International 2018, Article ID 8545347, 27 pages; https://doi.org/10.1155/2018 /8545347. As shown for example in Table 3 of Konoshenko *et al.,* the selection of the method for isolating the extracellular vesicles allows a preselection of their size. In order to obtain extracellular vesicles > 200 nm and in particular > 300 nm preferably the systems ExoQuick^{™} (System Biosciences, USA), see also the Examples, and PROSPR (PRotein Organic Solvent PRecipitation) are used, the latter in particular for analytical purposes.

The ability to form extracellular vesicles has long been known for various microorganisms (Lee et al., Proteomics 9 (2009), 5425-5436) and this property has also been described for plant cells, see for example Ju et al., Mol. Ther. 21 (2013), 1345-1357. In principle, it would therefore be possible that the vesicles of the invention in the fermentation product are secreted either only by cells of the plant raw material or only by the microorganisms present, wherein in the latter case it can be assumed that the vesicles absorb compounds originating from plant cells and formed during fermentation, respectively. Without intending to be bound to a certain theory, it is assumed in the present case that the vesicles of the invention originate both from the plant raw material, *i.e.* from the plant cells as well as from the cells of the microorganisms. As described above, the different types of vesicles differ *inter alia* in their size, but also in their characteristic cellular genesis, and can therefore have different protein and nucleic acid compositions due to their different formation. In particular, there are also differences in lipid composition between extracellular vesicles derived from animal, plant or bacterial cells. Methods for the analysis of compositions of the vesicles are described in detail in Müller, J. Bioanal. Biomed 4 (2012), 4 and *inter alia* a method for determining the lipid composition is also described in Ju et al., Mol Ther 21 (2013), 1345-1357. Accordingly, in the invention, the vesicles are characterized by the presence of vesicles and/or membrane components of microbial and plant origin.

Experiments performed in accordance with the present invention confirmed that extracellular vesicles isolated from raw plant material prior to fermentation (A) and from bacteria fermented with fructose but without further ingredients (B) as well as from the fermentation product of raw plant with bacteria (C) each show a unique size distribution with the extracellular vesicles from the fermentation product of the raw material (C) not just displaying a mere additive but in addition or alternative an intermediate size distribution; see Example 3 and Fig. 2. These data show that extracellular vesicles derived from the fermentation product of plant raw materials fermented by microorganisms such as bacteria are of microorganism and plant origin. Accordingly, extracellular vesicles (EV) derived from a fermentation product of natural raw materials such as plant raw materials are clearly distinguishable from EV derived from plant material and the microorganisms used for the fermentation as well as from the simple mixture thereof. Corresponding analyses may be preferably made in accordance with the appended Examples and the methods described therein.

Accordingly, in one embodiment of the present invention, the extracellular vesicles derived from the fermentation product of plant raw material are different, *e.g.* in their size, from the extracellular vesicles obtainable from plant raw material prior to fermentation and the bacteria used for the fermentation process.

The present invention relates to extracellular vesicles which are derived from a fermentation product of plant raw materials comprising lemons, pitted dates, figs, walnuts, coconuts, soybeans, onions, mung bean sprouts, celery, artichokes, millet, and peas and wherein the fermentation comprises
(a) the production of a first ferment extract or ferment powder;
(b) subj ecting a portion of the first ferment extract or ferment powder to at least one further fermentation; and
(c) mixing the portion/s of the further fermentation/s with the remaining portion of the first fermentation extract or fermentation powder,
wherein the microorganisms consist of lactic acid bacteria, namely of *Lactobacillus,* and wherein the size of the EV is between 150-210 nm, 240-300 nm, 350-410 nm and 575-635 nm, preferably between 175-185 nm, 265-275 nm, 375-385 nm and 600-610 nm.

Preferably, the microorganisms carrying out the fermentation are selected from the group consisting of: *Lactobacillus acidophilus, Lactobacillus amylolyticus, Lactobacillus amylovorus, Lactobacillus brevis, Lactobacillus bulgaricus, Lactobacillus casei (including Shirota), Lactobacillus crispatus, Lactobacillus farraginis, Lactobacillus gasseri, Lactobacillus helveticus, Lactobacillus iners, Lactobacillus jensenii* , *Lactobacillus johnsonii, Lactobacillus paracasei, Lactobacillus parafarraginis, Lactobacillus plantarum, Lactobacillus reuteri, Lactobacillus rhamnosus, Lactobacillus salivarius, Lactobacillus sporogenes, Lactobacillus zeae,* and/or mixtures thereof, wherein the microorganisms are either already present in/on/at the plant raw materials (spontaneous fermentation) or are added externally to the raw materials.

In one embodiment of the present invention, fermentation is carried out by only one of the above-mentioned species of microorganisms. In another embodiment of the present invention, the fermentation is carried out by a combination of one or more of the above-mentioned species of microorganisms, wherein the microorganisms either carry out the fermentation together or are added successively to the raw material at different fermentation stages.

In accordance with the present invention, the fermentation is carried out by microorganisms of the genus *Lactobacillus.* In a preferred embodiment, the fermentation is carried out either by microorganisms of the species *Lactobacillus paracasei* alone or by *Lactobacillus paracasei* in combination with *Lactobacillus rhamnosus,* wherein *Lactobacillus paracasei* and *Lactobacillus rhamnosus* are preferably added to the plant raw material at different fermentation stages.

In addition to the preferred fresh and freshly stored foodstuffs as characterized in the claims, additionally processed foodstuffs and luxury foodstuffs as well as their wastes as raw material sources for fermentation and the fermentation product are considered as natural raw materials and raw materials, from which vesicles of the invention can be isolated, such as in particular those from the preparation of foodstuffs such as cellulose and natural fibers, spent grain, pomace, oat husks, beet pulp, cocoa bean husks, boiled potatoes, boiled rice, potato residues, molasses, cocoa bean, fruit and vegetable husks, fruit stones, malt spent grains and other vegetable wastes which are used as permissible raw materials for the production of quality compost. Accordingly, the present invention also makes a contribution to the utilization of residues from the production of food, luxury foodstuffs and animal feed.

Of course, additional substances can be added to the raw material, such as various plant extracts, plant concentrates, vitamins, minerals, trace elements, flavoring substances and/or other organic compounds. Thus the raw material and the fermentation product, respectively, in a further embodiment comprise additional extracts, concentrates, vitamins, minerals, trace elements, flavoring substances and/or other organic compounds. In addition or alternatively, the raw material and fermentation product, respectively, is free or substantially free of coloring agents, preservatives, alcohol, sugar, in particular lactose, gluten and milk. In particular, for alcohol 'substantially free' means that less than 2%, preferably less than 1.5%, more preferably less than 1.2% and most preferably less than 0.9% alcohol is present in the raw material and fermentation product, respectively. With respect to lactose, 'substantially free' means that less than 1%, preferably less than 0.5%, and most preferably between 0.1% and 0.3% lactose is present in the raw material and fermentation product, respectively.

Extracellular vesicles of different sizes were found in the fermentation product of plant raw materials. The purification and quantification including size determination of the vesicles of the invention from the fermentation product of plant raw materials on a small scale was carried out as described in the Examples. Vesicles with a size between 30 nm and 1000 nm were found. In particular, as shown in Example 2, using the ExoQuick technology vesicles of four different sizes were found, *i.e.* vesicles of about 181 nm, about 271 nm, about 378 nm, and about 604 nm were found in a representative measurement.

The vesicles of the invention have a size of
(i) between 150 nm and 210 nm, preferably between 160 nm and 200 nm, more preferably between 170 nm and 190 nm, even more preferably between 175 nm and 185 nm and most preferably they have a size of 181 nm;
(ii) between 240 nm and 300 nm, preferably between 250 nm and 290 nm, more preferably between 260 nm and 280 nm, even more preferably between 265 nm and 275 nm and most preferably they have a size of 271 nm;
(iii) between 350 nm and 410 nm, preferably between 360 nm and 400 nm, more preferably between 370 nm and 390 nm, even more preferably between 375 nm and 385 nm and most preferably they have a size of 378 nm; and
(iv) between 575 nm and 635 nm, preferably between 585 nm and 625 nm, more preferably between 595 nm and 615 nm, even more preferably between 600 nm and 610 nm and most preferably they have a size of 604 nm.

In the present invention, the fermentation product of plant raw materials comprises vesicles of the invention in all four sizes of (i) - (iv) described.

Accordingly, the present invention also provides compositions which contain the vesicles of the invention in the above-mentioned combination of sizes.

In a preferred embodiment, the vesicles of the invention of the size of (i) to (iv) are present in the following distribution:
(i) 30-40%, preferably about 35±2%;
(ii) 25-35%, preferably about 29±2%;
(iii) 25-35%; preferably about 30±2%; and
(iv) 2.5-10%, preferably about 6±2%; see also the Examples.

Extracellular vesicles usually contain *inter alia* proteins, lipids, nucleic acids and membrane receptors. These can be detected by means of standardized procedures known to the person skilled in the art. Experiments showed that nucleic acids can be isolated from the vesicles of the invention. In particular, from 10 ml of the fermentation product of plant raw materials, it was possible to isolate 1.2 µg DNA and 13 µg RNA; see Example 5. Accordingly, in one embodiment more than 0.1 µg DNA and/or more than 1 µg RNA can be isolated from the vesicles isolated from 1 ml of the fermentation product.

The analysis of the components of extracellular vesicles is usually preceded by a step of their isolation. Methods for the isolation of exosomes are summarized, for example, in the dissertation "Isolierung und Charakterisierung funktioneller Exosomen durch sequentielle Filtration" by Heinemann 2016, wherein a promising method with respect to the isolation of exosomes is a method developed by Lamparski *et al.* (Lamparski et al., Journal of Immunological Methods 270 (2002), 211-226). This method is based on a combination of tangential filtration (TFF) and subsequent ultracentrifugation over a deuterium sucrose gradient. This method represents a significant advance over other methods as it enables clinical grade exosomes to be isolated for the first time. These particularly pure exosome preparations are already used in clinical studies on patients, for example for experimental immunotherapy of cancer patients (Escudier et al., Journal of Translational Medicine 3 (2005), 10 and Morse et al., Journal of Translational Medicine 3 (2005), 9).

In general, proteins can be detected by electrophoresis, mass spectrometry, flow cytometry and proteomics. Nucleic acids can be detected by PCR, microarray analysis, deep sequencing and next-generation sequencing. Lipids can be detected by gas chromatography (GC), mass spectrometry or HPLC. Such detection methods are listed among others in Zaborowski et al., BioScience 65 (2015), 783-797. A general method for isolation and further methods for characterization of exosomes and microvesicles are further described in Müller, J Bioanal Biomed 4 (2012), 4.

Thus, the present invention provides extracellular vesicles according to the claims comprising an active ingredient of interest, wherein said active ingredient is preferably selected from the group consisting of proteins, lipids, nucleic acids and membrane receptors of the cell of origin.

In this context, the vesicles of the invention according to the claims preferably contain biologically active ingredients and active ingredient, respectively, which have an effect on animal and human cells and on the animal and human organism, respectively, in general. A method for detecting biological activity of the vesicles of the invention is described in Example 4. In this context, it has been shown in Example 4 that the extracellular vesicles of the present invention advantageously exhibit a positive effect on the non-mitochondrial oxygen consumption which represents one component of the bioenergetics health index (BHI); see Fig. 3. For this reason it can be concluded that the vesicles of the invention have a positive effect on the BHI which is considered as a biomarker for assessing patient health with both prognostic and diagnostic value (Chacko et al., Clin. Sci. 127 (2014), 367-373).

The proteins can be selected from the group consisting of heat shock proteins / chaperones, major histocompatibility complex (MHC) proteins, endosomal sorting complexes required for transport (ESCRT) proteins, matrix metalloproteinases (MMP), as well as enzymes such as phospholipases, fatty acid synthases, integrins, glyco- and phosphoproteins and selectins. Lipids may be selected from the group consisting of gangliosides, sphingomyelin and unsaturated fats. Nucleic acids may be selected from the group consisting of ncRNA, rRNA, mRNA, miRNA, tRNA, and dsDNA, sDNA. Membrane receptors include, for example, the major histocompatibility complex (MHC) proteins. It should be noted that the above lists are not complete and are not intended to limit the present invention.

In addition, it can be assumed, however, without being bound to a certain theory, that the vesicles of the invention according to the claims also include other biologically active ingredients, especially secondary plant metabolites. This can be justified by the fact that, as described above, the extracellular vesicles can also originate from eukaryotic, here plant cells, which comprise the secondary metabolites mentioned.

These secondary plant metabolites include, but are not limited to *inter alia* phenolic compounds such as simple phenols, polyphenols including flavonoids such as flavonoids such as flavonols, flavanones, flavones, flavan-3-ols, anthocyanins, anthocyanidins, isoflavones, dihydroflavonols, chalcones and cumestans, phenolic acids, hydroxycinnamic acids, lignans, tyrosol esters, stilbenoids, hydrolysable tannins, carotenoids such as carotenes and xanthophylls, monoterpenes, saponins, xanthones, phenylpropanoids, stilbenes and their glycosides, isoprenoid compounds including terpenes, steroids and their glycosides, betalaines such as betacyanines and betaxanthines, carotenoids such as carotenes and xanthophylls, storage lipids, alkaloids such as caffeine and nicotine, amino acids such as alliine or canavanine, lipids such as phytosterols, tocopherols and omega-3,6,9 fatty acids. Such secondary plant metabolites can be detected using standardized HPLC and GC analyses with and without coupled mass spectrometry.

However, without being bound to any particular theory, it can reasonably be expected that the extracellular vesicles of the invention contain active ingredients that have a positive effect on health after ingestion in a human or animal subject.

Initially, it is known that exosomes can fuse with the cytoplasmic membrane of a target cell. This fusion turns exosomal proteins into new components of the cell surface. For example, receptors can be transferred intercellularly. One consequence of this is that cells modulated in this way can be activated by new bioactive ligands. Through fusion with the cell, the nucleic acids contained in the exosome (e.g. mRNAs, miRNAs) also become part of the cytoplasm of the cell. Subsequently, the genome of the cell can be epigenetically modulated as a result of this transfer (Valadi et al., Nat Cell Biol 9 (2007), 654-659). This can be done, for example, by silencing or enhancing certain gene segments with exosomal miRNAs. In addition, exosomally transported mRNAs can be translated directly from the target cell (Deregibus et al., Blood 110 (2007), 2440-2448). Exosomes can also cause effects on target cells without fusion with the cell membrane. The activation of cell membrane receptors on the target cell can lead to the activation of intracellular signaling cascades with specific consequences depending on the signaling cascade.

It is also known from the literature that extracellular vesicles have several properties of an efficient drug delivery system, so that potential active ingredients can be absorbed more quickly, *i.e.* the bioavailability of active ingredients encapsulated in extracellular vesicles is increased. For example, despite numerous bioactive and therapeutic properties, curcumin has only limited pharmaceutical benefit due to its poor water solubility and stability and low systemic bioavailability. By encapsulation in milk exosomes the stability of curcumin in PBS could be increased, it survived hard digestion processes and crossed the intestinal barrier as free curcumin. The encapsulation of curcumin in the exosomes thus increased its stability, solubility and bioavailability (Vashisht et al., Appl. Biochem. Biotechnol. 183 (2017), 993-1007).

Accordingly, it is reasonable to expect that active ingredients encapsulated in extracellular vesicles of the invention, *i.e.* those derived from a fermentation product of plant raw materials according to the claims, also exhibit increased bioavailability, stability and solubility and can thus be made available to the human or animal organism.

As already described above, it can be expected that the vesicles of the invention in the fermentation product of plant raw materials originate from microorganism cells and plant cells.

In the case of vesicles derived from bacteria, it has already been shown that they have a positive effect on human health. For example, vitamin K2 (menaquinone) is uniquely produced by bacteria, including *Bacillus subtilis,* certain lactic acid bacteria and propionibacteria, and acts in the human body as a carboxylase cofactor for protein maturation, which is involved in many essential physiological processes (Liu *et al.,* 49 (2018), 179-184). Furthermore, it was shown that extracellular vesicles produced by microbiome-associated bacteria can cross the epithelial barrier of the human mucosa and interact with immune cells (Zhang et al., PLoS One 8 (2013), e77966).

Concerning extracellular vesicles from plant cells, *e.g.* Ju et al. Mol. Ther. 21 (2013), 1345-1357 have shown that exosome-like vesicles could be isolated from grapes and that these vesicles triggered the proliferation of intestinal stem cells after oral administration and showed therapeutic effects in an experimental model of colitis in mice. Mu *et al.* (2014) have shown that exosome-like nanoparticles are absorbed by the intestine and induced the expression of genes that are crucial for the maintenance of intestinal homeostasis (anti-inflammatory cytokines, antioxidants, etc.) (Mu et al., Mol. Nutr. Food Res. 58 (2014), 1561).

Furthermore, phytochemicals as bioactive plant substances, which are assumed to be also present in extracellular vesicles, have attracted much attention as preventive and therapeutic ingredients against cancer by demonstrating that phytochemicals regulate the expression of microRNAs (miRNAs/miRs), which are functionally involved in cancer pathobiology. An overview is given in the book "Mitochondria as Targets for Phytochemicals in Cancer Prevention and Therapy", Chandra, Dhyan (ed.) (2013), 187-206.

Further effects of plant secondary metabolites are described in numerous publications. For example, saponins, which are mainly found in legumes, oats and some vegetable species, and flavonoids, which are present in almost all plants, have anti-inflammatory effects (Watzl and Leitzmann: Bioaktive Substanzen in Lebensmitteln. 3rd, unchanged edition). Hippokrates, Stuttgart 2005, ISBN 3-8304-5308-6; Watzl and Rechkemmer: Basiswissen aktualisiert: Flavonoide. In: Ernährungs-Umschau. 48, Issue 12, 2001. Carotenoids such as alpha-carotene, beta-carotene and beta-cryptoxanthin, which give orange and yellow fruits and vegetables their colour, are converted in the body to vitamin A, which is important for a healthy immune system as well as for healthy skin and mucous membranes. Phytin from cereals has its effect in the regulation of the blood sugar level.

Thus, in one embodiment of the invention, such plant materials containing the active ingredient of interest can be increasingly added to the plant raw material according to the claims in order to produce compositions with the corresponding effect of interest. It is particularly interesting that the vesicles of the invention originate from a fermentation product according to claim 1 of plant raw material, wherein the raw materials comprise lemons, pitted dates, figs, walnuts, coconuts, soybeans, onions, mung bean sprouts, celery, artichokes, millet, and peas.

For example, more orange and yellow fruits and vegetables can be added to the plant material in order to accumulate carotenoids in the vesicles of the invention and accordingly provide compositions that have a positive effect on the immune system. Such approaches are conceivable in a wide variety of combinations and are covered by this invention.

Thus, vesicles of the invention or the composition of the invention are provided for use as drugs. According to the present invention, the fermentation of the raw materials comprises the production of a first ferment extract or ferment powder, subjecting a portion of the first ferment extract or ferment powder to at least one further fermentation and mixing this portion with the remaining portion of the first ferment extract or ferment powder, *i.e.* the fermentation is a cascade fermentation.

Fermentation according to the present invention is carried out in the presence of microorganisms of the genus *Lactobacillus,* in particular microorganisms of the species *Lactobacillus paracasei* and/or *Lactobacillus rhamnosus.*

With regard to the raw materials, the plant raw materials described above are used in the combinations described above. Before fermentation, they are shredded in the usual way, e.g. by cutting and/or shredding. In another embodiment of the method of the invention, plant raw material containing the active ingredient of interest is fermented or the active ingredient is added to the raw material and/or fermentation product.

Cascade fermentation comprises the fermentation of plant raw materials in the presence of microorganisms to produce a ferment extract or ferment powder and the subsequent subjecting of a portion of the ferment extract or ferment powder to at least one further fermentation. For this purpose, preferably up to one third of the previous ferment extract, which has been cleaned by centrifugation and filtration and dried if necessary in the case of the ferment powder, is separated and fermented again. In the further course of the description, for the sake of simplification, only a ferment extract is mentioned, which of course also means dried ferment powder.

Partial fermentation is normally carried out at a temperature in the range 20°C to 35°C, the temperature depending on the microorganism used. In general, the temperature is between 28°C and 32°C. The period of incubation also depends on the microorganism used. Normally the period of the partial fermentation is shorter than that of the first batch, *i.e.* the initial fermentation of the mixture of naturally occurring raw materials. The partial ferment is then cleaned like the first ferment extract by centrifugation and filtration using conventional methods. The cleaned partial ferment is then mixed with the remaining part of the first ferment extract. Preferably a propagate of microorganisms is added to the combined ferment extracts.

It has proved advantage to carry out two partial fermentations. This method variant is designed in such a way that again a portion of the fermentation product is separated, which is subjected to a further second fermentation. This portion is then mixed with the remaining portion. Any number of partial fermentations can be carried out.

The fermentation according to the present invention is carried out in the presence of microorganisms of the genus *Lactobacillus,* in particular microorganisms of the species *Lactobacillus paracasei* and/or *Lactobacillus rhamnosus,* the former preferably being used in the main fermentation and after mixing the first ferment extract with the extract from the main fermenter, and the latter preferably in the first partial fermentation. With regard to the quantities of the microorganisms used and the order of addition in the individual ferment extracts, explicit reference is made to the European patents EP 1 153 549 B1 and EP 2 560 506 B1.

The raw materials used are the plant raw materials already described above in the combinations described above. Before fermentation, they are shredded in the usual way, e.g. by cutting and/or shredding. In another embodiment of the method of the invention, plant raw material containing the active ingredient of interest is fermented or the active ingredient is added to the raw material and/or fermentation product. With regard to the quantities of raw materials, explicit reference is made to the European patents EP 1 153 549 B1 and EP 2 560 506 B1.

Likewise, regarding the exact fermentation conditions, *inter alia* regarding the supply of nutrient solutions and other additives, the oxygen values as well as the various possible method variants A and B reference is made to the European patent EP 2 560 506 B1. Furthermore, the fermentation of plant raw materials is described in detail in Example 1 of the present invention.

The recovery or enrichment of the vesicles of the invention from the fermentation product is preferably carried out by ultra-, density gradient centrifugation, electrophoresis, chromatography, column method, precipitation, lyophilization, drying, and/or nanofiltration. Methods for obtaining or enriching extracellular vesicles and exosomes, respectively, are described for example in EP 2 839 278 B1, WO 2013/158203 A1 or WO 2013/188832 A1.

A further embodiment of the present invention relates to a composition containing the extracellular vesicles of the invention and those produced by the method of the invention. Using the method described in the Examples, the number of vesicles in the fermentation product of plant raw materials was determined. For vesicles with a size of about 604 nm a number of 0.25 × 10¹⁰ vesicles/ml was determined, for vesicles with a size of about 378 nm a number of 1.26 × 10¹⁰ vesicles/ml was determined, for vesicles with a size of about 271 nm a number of 1.25 × 10¹⁰ vesicles/ml was determined and for vesicles with a size of about 181 nm a number of 1.48 × 10¹⁰ vesicles/ml was determined. Accordingly, the present invention provides compositions in which the concentration of the vesicles of the invention is greater than 1 × 10⁸ /ml, particularly preferably greater than 1 × 10⁹ /ml and especially preferably greater than 1 × 10¹⁰ /ml.

The size of the EV in the composition of the present invention is between 150-210 nm, 240-300 nm, 350-410 nm and 575-635 nm, preferably between 175-185 nm, 265-275 nm, 375-385 nm and 600-610 nm.

The composition of the invention is a natural product, *i.e.* a biological product and can be used for internal and external administration. For internal administration, it is usually given orally as a food or food supplement or drug. It may be administered in the form of a liquid. It is also possible to form a powder or lyophilizate thereof, which can be dissolved in water or another liquid if required. It is also advantageous to administer the natural product of the invention in the form of tablets, pastilles, granules, ampoules, drops or sprays. Accordingly, the composition of the invention can be a natural product, drug, food for special medical purposes (balanced diets), dietary food, food supplements, cosmetic or body care product. Where appropriate, the composition of the invention comprises a pharmaceutically or physiologically acceptable carrier and/or stabilizer. Such are known to the skilled artisan.

The natural product produced according to the invention can also be applied externally to the skin. It is also possible to use the natural product produced according to the invention as a cosmetic product, for example incorporated in creams, ointments and foams, with which it is applied directly to the skin. The cosmetic products have moisturizing properties and act as anti-aging agents.

Furthermore, the present invention comprises the use of the vesicles of the invention or vesicles produced according to the method of the invention or the composition of the invention in the manufacture of foods, food supplements, drugs and/or in a cosmetic or personal care product.

The above-mentioned methods and procedures for the characterization of the extracellular vesicles of the invention can be *inter alia* used to analyze their quantity, quality, *i.e.* their surface structure and their ingredients. In particular, the extracellular vesicles originating from the microorganisms used in fermentation and/or the plant raw materials can be examined, from which a definite and targeted therapeutic benefit can be derived. For example, it can be determined that the extracellular vesicles obtained by means of fermentation with the addition of microorganism X and/or with the addition of raw material Y have a benefit in the treatment of the disease Z due to their number, quality and ingredients. The knowledge that extracellular vesicles are present in the fermentation product of plant raw materials can also be used, for example, for the quality control of food fermentations. It has already been shown that during cell death more vesicles were released into the medium, so that the amount of extracellular vesicles present can provide information on the state of the bioprocess (Zavec et al., Biotechnol J. 11 (2016), 603-609).

Processes and methods for the isolation of extracellular vesicles are known in the prior art, such as the ExoQuick^{™} System (ExoQuick-TC) from Biosciences (SBI), Palo Alto, CA, USA, which is also used in the Examples. However, these have not yet been used for the isolation of extracellular vesicles with the characteristics shown in the Examples, in particular due to their origin from a fermentation product and the observed size distribution, which deviates considerably from the usually observed, essentially monotonous size in the range of about 50-200 nm. An exemplarily method for the insolation of EVs is characterized by
(a) contacting a sample of a fermentation product with a solution comprising at least one volume-excluding polymer to form a polymer-microvesicle complex; and
(b) separating the polymer-microvesicle complex from the solution;
wherein the polymer preferably is polyethylene glycol, dextran, dextran sulfate, dextran acetate, polyvinyl alcohol, polyvinyl acetate or polyvinyl sulfate.

The following examples illustrate the invention.

### EXAMPLES

### Example 1: Production of a fermentation product from plant raw materials

For the production of the fermentation product, 70 to 100 kg of plant raw materials in the form of about 25% lemons, about 18-20% pitted dates, about 18-20% figs, about 8-10% walnuts, about 6% coconuts, about 6% soybeans, about 6% onions, about 2.5% mung bean sprouts, about 2.5% celery, about 1% artichokes, about 0.75% millet, about 0.75% peas and 80 to 100 L water.

Initially, three different batches were prepared.
1) The soybeans and peas were cooked for 20 min and the millet for 10 min.
2) The fresh ingredients were washed with hot water between 60°C and 80°C and crushed.
3) A bacterial culture (300 to 500 g *Lactobacillusparacasei*) was produced including about 1 kg fructose.

The three batches were then transferred to a 10,000 litre bioreactor and 250 to 350 kg of lactose were added as fermentation starters.

The first fermentation was carried out at a temperature of 30°C to 32°C for more than 2 weeks with an oxygen concentration of about 0.2 g/kg and a CO₂ concentration of about 1.8 g/kg. Subsequently, a first portion was removed from the bioreactor and the pH value of this portion was determined. A second bacterial culture *Lactobacillus rhamnosus* was added to this portion. The second fermentation was carried out at 30°C to 32°C for about 1/3 of the time of the first fermentation. A second portion was then removed from the bioreactor and the pH value of this portion was determined. A bacterial culture (*Lactobacillus paracasei*) plus 1 kg fructose was added to this portion.

The third fermentation was carried out at 30°C to 32°C for a similar period as the second fermentation. Three portions were mixed in the bioreactor and then a third portion was removed from the bioreactor.

The portion was purified by sieving and subsequent centrifugation. The purification process was carried out until the desired degree of turbidity was achieved.

### Example 2: Quantification and size determination of extracellular vesicles and exosomes

The isolation of the extracellular vesicles and exosomes from the fermentation product of plant raw materials described above was performed using the commercially available ExoQuick^{™}-TC exosome isolation reagent from System Biosciences (CAT EXOTC10A-1) according to the manual.

For the subsequent quantification and size determination, the NanoSight^{™} technique was used, by means of which small particles, which can be as small as 10 nm, can be visualized, characterized and measured in a solution. The NanoSight^{™} technology comprises a scientific camera, a microscope and a sample acquisition unit. The latter uses a laser diode to illuminate particles in liquid suspension that are held in a flow chamber within the unit or advanced there through. The device is used in conjunction with a computer control unit that operates a customized Nanoparticle Tracking (NTA) software package. With NTA, the particles are automatically tracked and sized. The results are displayed as a frequency distribution graph and exported in various user-selected formats, including spreadsheets and video files. In addition, information-rich video clips can be captured and archived for future reference and alternative analyses. NTA enables the determination of a size distribution profile of small particles with a diameter of about 10-1000 nm in liquid suspension.

The results of the described analysis showed that the fermentation product of plant raw materials contains extracellular vesicles in different sizes and amounts. In detail, it could be shown that about 0.25 × 10¹⁰ vesicles/ml with a size of 604 nm were present in the fermentation product, corresponding to a proportion of 5.8%; that about 1.26 × 10¹⁰ vesicles/ml with a size of 378 nm were present in the fermentation product, corresponding to a proportion of 29.6%; that about 1.25 × 10¹⁰ vesicles/ml with a size of 271 nm were present in the fermentation product, corresponding to 29.2%; that about 1.48 × 10¹⁰ vesicles/ml with a size of 181 nm were present in the fermentation product, corresponding to 34.7%. The results are summarized in Table 1 and the results of a representative measurement are shown in Fig. 1.

**Table 1: Diameter, number per milliliter and percentage share of four different fractions of extracellular vesicles detected in the fermentation product of plant raw materials.**

| **Size of vesicles** | **Number of vesicles/ml** | **Percentage share** |
|---|---|---|
| 604 nm | 0.25 × 10¹⁰ | 5.8% |
| 378 nm | 1.26 × 10¹⁰ | 29.6% |
| 271 nm | 1.25 × 10¹⁰ | 29.2% |
| 181 nm | 1.48 × 10¹⁰ | 34.7% |

### Example 3: Extracellular vesicles isolated from raw plant and bacterial fermented material

In order to be able to draw conclusions regarding the origin of the extracellular vesicles present in the fermentation product of plant raw materials, extracellular vesicles isolated from raw plant material prior to fermentation (squeezed lemon juice) as well as bacteria fermented alone (7-day fermentation of *Lactobacillus paracasei* with fructose w/o further raw materials) and fermented plant material (7-day single fermentation of lemons and *Lactobacillus paracasei* under lab conditions) were analyzed and compared. The extracellular vesicles were isolated and analyzed as described in Example 2, *i.e.* using the ExoQuick^{™} system and NanoSight^{™} technology.

The results as depicted in Fig. 2 show that the single components of the fermentation product, *i.e.* plant raw materials (squeezed lemon juice) prior to fermentation (Fig. 2A) as well as fermented bacteria without further raw material added (apart from fructose) (Fig. 2B) and fermented plant raw material (Fig. 2C) contain extracellular vesicles in different sizes and amounts comparable to the results of the analyses depicted in Example 2. In particular, from squeezed lemon juice extracellular vesicles were isolated with a size between about 50 nm and 500 nm with peaks at 80 nm, 128 nm, 156 nm, 240 nm, 289 nm, 347 nm, 463 nm, and 500 nm (Fig. 2A). The fermentation product of *Lactobacillus paracasei* with fructose contained extracellular vesicles between about 100 nm and 1200 nm with peaks at 193 nm, 253 nm, 398 nm, 468 nm, 547 nm, 668 nm, 826 nm, and 1071 nm (Fig. 2B). From the fermentation product of lemons fermented by *Lactobacillus paracasei* extracellular vesicles were isolated with a size between about 100 nm and 1200 nm with peaks at 125 nm, 188 nm, 272 nm, 426 nm, 574 nm, 721 nm, 771 nm, 894 nm, 1046 nm, and 1200 nm (Fig. 2C).

### Example 4: Determination of the biological activity of extracellular vesicles

Peripheral blood leukocytes shall be used to perform tests on the biological activity of extracellular vesicles derived from the fermentation product of plant raw materials.

For expression analysis of PGC-1-alpha, Nrf-2 and rhodanase, analysis of ATP production and viability, human peripheral blood leukocytes (PBMC) are first isolated from the whole blood of a voluntary donor and adjusted to a defined cell number. The PBMCs are incubated with three different concentrations of H₂O₂ for 48 h to 72 h at 37°C and 5% CO₂. Incubation occurs in the absence or presence of different concentrations of the extracellular vesicles. This is followed by expression analysis of PGC-1-alpha, Nrf-2 and rhodanase, analysis of ATP production and viability. The biological activity of EV is indicated by an increased expression and activation, respectively, of PGC-1alpha, Nrf-2 and/or rhodanase.

In another experiment, human peripheral blood leukocytes (PBMC) are isolated from the whole blood of a volunteer donor and adjusted to a defined cell number. The cellular bioenergetic health index (BHI), which includes the following parameters, is then analyzed:
a) mitochondrial and non-mitochondrial oxygen consumption
b) mitochondrial and non-mitochondrial ATP production
c) proton leak
d) mitochondrial reserve respiratory capacity
e) maximum possible mitochondrial oxygen consumption rate
f) Crabtree effect
g) Warburg effect.

Methods for the detection of the biological activity of the extracellular vesicles on the energy production of the cells via mitochondrial activity increase are known to the skilled person and described for example in Chacko et al., Clin. Sci. 127 (2014), 367-373; Dinkova-Kostova and Abramov, Free Radic. Biol. Med. 88 (2015), 179-188; Mimoun et al., Antioxide. Redox signal. 17 (2012), 1-10; and Puigserver, Int. J. Obes. 29 (2005),Suppl 1: S5-9. The analysis of the listed parameters is performed in the absence or presence of different concentrations of the extracellular vesicles. The biological activity of the EV is indicated by an increase in the cellular bioenergetic index (BHI). Alternatively, the investigations can be performed with CACO-2 cells as described in Li et al., BMC Microbiology 17 (2017), 66.

The non-mitochondrial oxygen consumption rate (OCR) was determined as one parameter of the BHI. The following ratios of PBMC to EVs were applied: 1:1,000; 1:100; 1:10; 1:1; 1:0.1; 1:0.01; and 1:0 (w/o), wherein 2.5 × 10⁵ PBMC were used and the initial concentration of the EVs was 4.21 × 10¹⁰ particles/ml. The non-mitochondrial OCR is an index of oxygen-consuming processes that are not mitochondrial. In leucocytes, non-mitochondrial OCR is typically increases in the presence of stressors including reactive nitrogen species (RNS) and reactive oxygen species (ROS) and is regarded as negative indicator of bioenergetic health (Chacko et al., Clin. Sci. 127 (2014), 367-373).

The results of the treatment of PBMC with different concentrations of EVs is shown in Fig. 3. The proportion of non-mitochondrial respiration on the overall respiration of untreated cells (w/o) is set to 100%. The data indicate that treatment of the cells with extracellular vesicles reduces non-mitochondrial respiration. Thereby, a reciprocal dose/effect dependency is observed.

### Example 5: Isolation of nucleic acid from extracellular vesicles

In order to determine that the extracellular vesicles of the invention contain biomolecules such as nucleic acids, DNA and RNA was isolated from the extracellular vesicles of the invention using the Qiagen DNeasy Kit. Thereby, 1.2 µg DNA and 13 µg RNA could be isolated from extracellular vesicles obtained by the procedure as described in Example 2 from 10 ml of the fermentation product.

## Claims

1. Extracellular vesicles (EV) derived from a fermentation product of natural raw materials and microorganisms, wherein the natural raw materials are plant raw materials and comprise lemons, pitted dates, figs, walnuts, coconuts, soybeans, onions, mung bean sprouts, celery, artichokes, millet, and peas, and wherein the EV are of plant and microbial origin, wherein the fermentation of the natural raw materials comprises
(a) the production of a first ferment extract or ferment powder;
(b) subjecting a portion of the first ferment extract or ferment powder to at least one further fermentation; and
(c) mixing the portion/s of the further fermentation/s with the remaining portion of the first fermentation extract or fermentation powder,
wherein the microorganisms consist of *Lactobacillus,* and wherein the size of the EV is between 150-210 nm, 240-300 nm, 350-410 nm, and 575-635 nm, preferably between 175-185 nm, 265-275 nm, 375-385 nm, and 600-610 nm, and wherein the size of the EV is measured as described in Example 2 of the description.

2. The EV according to claim 1 comprising an active ingredient of interest, preferably selected from the group consisting of a phytochemical active ingredient, nucleic acid molecules, proteins, lipids and membrane receptors.

3. A method for the preparation or enrichment of extracellular vesicles (EV) as defined in claim 1 or 2 comprising steps (a), (b) and (c) of claim 1 and comprising a step of recovering or enriching the EV from the fermentation product, optionally wherein the active ingredient is added to the raw material and/or fermentation product.

4. A composition comprising EV according to claim 1 or 2 or obtainable by the method of claim 3, preferably wherein the concentration of the extracellular vesicles is greater than 1 × 10⁸ /ml, more preferably greater than 1 × 10⁹ /ml and even more preferably greater than 1 × 10¹⁰ /ml.

5. The composition according to claim 4, preferably in liquid, powder or lyophilized form, which is a natural product, drug, food, food supplement, cosmetic or personal care product, optionally wherein the composition comprises a pharmaceutically or physiologically acceptable carrier and/or stabilizer.

6. Use of EV according to claim 1 or 2 or obtainable by the method according to claim 3 or a composition according to claim 4 or 5 in the manufacture of food, food supplement and/or in a cosmetic or personal care product.

7. EV according to claim 1 or 2 or obtainable by the method according to claim 3 or a composition according to claim 4 or 5 for use as a drug.

## Patentansprüche

1. Extrazelluläre Vesikel (EV), die aus einem Fermentationsprodukt von natürlichen Rohstoffen und Mikroorganismen stammen, wobei die natürlichen Rohstoffe pflanzliche Rohstoffe sind und Zitronen, entsteinte Datteln, Feigen, Walnüsse, Kokosnüsse, Sojabohnen, Zwiebeln, Mungobohnensprossen, Sellerie, Artischocken, Hirse und Erbsen umfassen, und wobei die EV pflanzlichen und mikrobiellen Ursprungs sind, wobei die Fermentation der natürlichen Rohstoffe
(a) die Herstellung eines ersten Ferment-Extrakts oder Ferment-Pulvers;
(b) Unterziehen eines Teils des ersten Ferment-Extrakts oder Ferment-Pulvers mindestens einer weiteren Fermentation; und
(c) Mischen des/der Teil/Teile der weiteren Fermentation/en mit dem verbleibenden Teil des ersten Fermentationsextrakts oder Fermentationspulvers, umfasst
wobei die Mikroorganismen aus *Lactobacillus* bestehen und wobei die Größe der EV zwischen 150-210 nm, 240-300 nm, 350-410 nm und 575-635 nm, vorzugsweise zwischen 175-185 nm, 265-275 nm, 375-385 nm und 600-610 nm, liegt und wobei die Größe der EV gemessen wird wie in Beispiel 2 der Beschreibung beschrieben.

2. EV nach Anspruch 1, umfassend einen Wirkstoff von Interesse, vorzugsweise ausgewählt aus der Gruppe bestehend aus einem phytochemischen Wirkstoff, Nukleinsäuremolekülen, Proteinen, Lipiden und Membranrezeptoren.

3. Verfahren zur Herstellung oder Anreicherung von extrazellulären Vesikeln (EV) wie in Anspruch 1 oder 2 definiert, umfassend die Schritte (a), (b) und (c) von Anspruch 1 und umfassend einen Schritt der Gewinnung oder Anreicherung der EV aus dem Fermentationsprodukt, wobei optional der Wirkstoff dem Rohmaterial und/oder Fermentationsprodukt zugesetzt wird.

4. Zusammensetzung, umfassend EV nach Anspruch 1 oder 2 oder erhältlich durch das Verfahren nach Anspruch 3, wobei vorzugsweise die Konzentration der extrazellulären Vesikel größer als 1 × 10⁸/ml, noch bevorzugter größer als 1 × 10⁹/ml und noch bevorzugter größer als 1 × 10¹⁰/ml ist.

5. Zusammensetzung nach Anspruch 4, vorzugsweise in flüssiger, pulverförmiger oder gefriergetrockneter Form, die ein Naturprodukt, ein Arzneimittel, ein Lebensmittel, ein Nahrungsergänzungsmittel, ein kosmetisches Produkt oder ein Körperpflegemittel ist, wobei optional die Zusammensetzung einen pharmazeutisch oder physiologisch verträglichen Träger und/oder Stabilisator enthält.

6. Verwendung von EV nach Anspruch 1 oder 2 oder erhältlich durch das Verfahren nach Anspruch 3 oder einer Zusammensetzung nach Anspruch 4 oder 5 bei der Herstellung von Lebensmitteln, Nahrungsergänzungsmitteln und/oder in einem Kosmetik- oder Körperpflegeprodukt.

7. EV nach Anspruch 1 oder 2 oder erhältlich durch das Verfahren nach Anspruch 3 oder eine Zusammensetzung nach Anspruch 4 oder 5 zur Verwendung als Arzneimittel.

## Revendications

1. Vésicules extracellulaires (EV) dérivées d'un produit de fermentation de matières premières naturelles et de micro-organismes, dans lesquelles les matières premières naturelles sont des matières premières végétales et comprennent des citrons, des dattes dénoyautées, des figues, des noix, des noix de coco, des fèves de soja, des oignons, des germes de haricots mungo, du céleri, des artichauts, du millet et des pois, et dans lesquelles les EV sont d'origine végétale et microbienne, dans lesquelles la fermentation des matières premières naturelles comprend
(a) une production d'un premier extrait de ferment ou d'une poudre de ferment ;
(b) une soumission d'une portion du premier extrait de ferment ou de la poudre de ferment à au moins une fermentation supplémentaire ; et
(c) un mélange de la ou les portion(s) de la ou des fermentation(s) supplémentaire(s) avec la portion restante du premier extrait de fermentation ou de la poudre de fermentation,
dans lesquelles les micro-organismes sont constitués de *Lactobacillus*, et dans lequel la taille des EV est comprise entre 150 à 210 nm, 240 à 300 nm, 350 à 410 nm et 575 à 635 nm, de préférence entre 175 à 185 nm, 265 à 275 nm, 375 à 385 nm et 600 à 610 nm, et dans lesquelles la taille des EV est mesurée comme décrit dans l'exemple 2 de la description.

2. EV selon la revendication 1, comprenant un ingrédient actif d'intérêt, de préférence choisi dans le groupe consistant en un ingrédient actif phytochimique, des molécules d'acide nucléique, des protéines, des lipides et des récepteurs membranaires.

3. Procédé de préparation ou d'enrichissement de vésicules extracellulaires (EV) selon la revendication 1 ou 2, comprenant les étapes (a), (b) et (c) de la revendication 1 et comprenant une étape de récupération ou d'enrichissement des EV à partir du produit de fermentation, facultativement dans lequel l'ingrédient actif est ajouté à la matière première et/ou au produit de fermentation.

4. Composition comprenant des EV selon la revendication 1 ou 2 ou pouvant être obtenus par le procédé de la revendication 3, de préférence dans laquelle la concentration des vésicules extracellulaires est supérieure à 1 × 10⁸/ml, de manière plus préférée supérieure à 1 × 10⁹/ml et de manière encore plus préférée supérieure à 1 × 10¹⁰/ml.

5. Composition selon la revendication 4, de préférence sous forme liquide, pulvérulente ou lyophilisée, qui est un produit naturel, un médicament, un aliment, un complément alimentaire, un produit cosmétique ou de soin personnel, facultativement dans laquelle la composition comprend un support et/ou un stabilisant pharmaceutiquement ou physiologiquement acceptable.

6. Utilisation d'EV selon la revendication 1 ou 2 ou pouvant être obtenues par le procédé selon la revendication 3 ou d'une composition selon la revendication 4 ou 5 dans la fabrication d'aliments, de compléments alimentaires et/ou dans un produit cosmétique ou de soin personnel.

7. EV selon la revendication 1 ou 2 ou pouvant être obtenues par le procédé selon la revendication 3 ou une composition selon la revendication 4 ou 5 pour une utilisation en tant que médicament.
